# EUROPEAN PATENT APPLICATION

(11) **EP 4 180 128 A1**
(43) Date of publication of application: **17.05.2023**
(21) Application number: 21386071.1
(22) Date of filing: 12.11.2021
(51) Int. Cl.: B01L 7/00, B01L 9/06

(54) **POCKET-SIZE DEVICE FOR ISOTHERMAL NUCLEIC ACID AMPLIFICATION**

(71) Applicant: BIOPIX DNA TECHNOLOGY P.C., 70013 Heraklion (GR)
(72) Inventor: Founta, Dimitra, 85300 Kos (GR); Pantazis, Alexandros, 48100 Preveza (GR); Fikas, Nikolaos, 56625 Thessaloniki (GR); Papadakis, Georgios, 71305 Heraklion, Crete (GR)
(74) Representative: Roukounas, Dimitrios

(57) **Abstract**

A pocket-size device (1) for isothermal nucleic acid amplification, which comprises an aperture (4) for receiving a reaction vessel (15) in which the nucleic acid amplification takes place and an electronic circuit (10). The electronic circuit (10) comprises a positive temperature coefficient thermistor (13) as heating element, a power port (9) and optionally a resistor (12).

## Description

### Technical field of the invention

The present invention relates to a pocket-size device for the performance of nucleic acid amplification at a constant elevated temperature.

### Background of the invention

Nucleic acid amplification is based on the ability of certain enzymes to amplify small quantities of nucleic acids in a sample and produce amplicons that can be subsequently, or in real-time, detected using various methods, such as fluorescence, or colorimetry. There are a number of different methods for the amplification of nucleic acids. Some of these methods, such as the polymerase chain reaction (PCR), require the exposure of the sample to repeated cycles of heating and cooling between 55°C and 95°C. Other methods, known as isothermal amplification methods, require the exposure of the sample to a constant elevated temperature, i.e. a temperature higher than the room temperature. A large number of isothermal amplification methods are known in the art and include the loop-mediated isothermal amplification (LAMP), the recombinase polymerase amplification (RPA), the rolling circle amplification (RCA), the isothermal identical multirepeat sequences amplification (isoIMRS) and others. The Loop mediated isothermal amplification (LAMP) method employs several primers (four to six) complementary to different regions of the analyzed DNA together with DNA polymerases with pronounced strand displacement activity. The use of several primers ensures high specificity of the reaction. LAMP is conducted at a constant temperature usually between 60-65°C and has an exponential character which allows for the generation of up to 10⁹ DNA copies within 15-60 min. The isothermal identical multirepeat sequences (iso-IMRS) amplification assay utilizes a Bst polymerase and a single pair of computationally identified repeated primers that bind to multiple loci across a genome for the exponential DNA amplification at a constant temperature. The recombinase polymerase amplification (RPA) method involves the formation of a complex between a recombinase enzyme and a forward and a reverse primer. This complex unwinds the DNA duplex and allows the amplification of the template in an exponential manner. RPA can be conducted at 22-45°C, although its optimal temperature range is 37-42°C. The rolling circle amplification (RCA) method is based on using a circular DNA formed by the interaction of the analyzed sequence with a single stranded DNA probe flanked by complementary sequences to the analyte. In the process of complex formation, the 5' and 3' ends of the probe are brought together and then ligated to form a circular molecule. The formed circular probe can hybridize with a primer that is elongated by DNA polymerase, resulting in the generation of a sequence consisting of numerous copies of the analyte DNA. Usually, RCA is carried out at 30-37°C and linear amplification at constant temperature takes from several hours to several days and results in the synthesis of multiple analyte copies.

A common feature among devices for the isothermal nucleic acid amplification of the state of the art is the requirement of an electronic circuit, which includes a heating element and a temperature sensor that regulates precisely the temperature, and a processing unit that controls the circuit. This is the main reason that is still expensive and complicated to perform molecular testing outside a diagnostics lab, such as for example, when a disposable rapid antibody/antigen test is used. Several attempts to create simple and low-cost molecular diagnostic devices have been previously developed, however most of them comprise complexed electronic circuits and microprocessors.

A. Ganguli et. al., Rapid isothermal amplification and portable detection system for SARS-CoV-2, PNAS, 2020, 117, 37, 22727-22735 discloses a microfluidic diagnostic cartridge combined with an instrument and a smartphone to detect the fluorescence emission from LAMP assays performed on the microfluidic cartridges. The instrument comprises four main parts to support optical, electrical, and heating components including a macro lens, a printed circuit board (PCB), a long-pass filter, eight LEDs, four short-pass filters, on-off switches and a self-regulating positive temperature coefficient heater.

N. Y. Jayanath et. al., Development of a portable electrochemical loop mediated isothermal amplification (LAMP) device for detection of hepatitis B virus, RSC Adv., 2018, 8, 34954-34959 discloses the development of a custom-made portable prototype device for real-time measurement of LAMP reactions using an electrochemical method. The system comprises a drop cell connector, a heat sink that is heated in a water bath at a preset temperature, a portable potentiostat and a microelectrode.

D. Kaygusuz et al., DaimonDNA: A portable, low-cost, loop-mediated isothermal amplification platform for naked-eye detection of genetically modified organisms in resource-limited settings, Biosensors and Bioelectronics, 2019, 141, 111409 discloses a portable device for colorimetric LAMP amplification and naked-eye results interpretation comprising the following electronic components; a Peltier effect heater, a temperature sensor, a printed circuit board (PCB), a thermocouple, a thermocouple digital converter and a power supply (12 V direct current, 5A). An Arduino Nano microcontroller was also used to control the electronics circuit, including a MOSFET and a transistor for switching and amplifying the signals to operate the Peltier.

US20200122142 A1 discloses a device for performing a biological assay such as DNA amplification and detecting an optical property of the biological sample under analysis. The employed components of the device include a heating element, a temperature sensor, a circuit board, a photosensor, light emitting elements, an electronic result displaying mechanism and a power supply.

A thermistor is a type of electrical resistor whose resistance depends on the temperature to a more significant extent than in standard resistors. There are two main categories of thermistors, those who exhibit a negative temperature coefficient (NTC), which means that they become better electrical conductors as the temperature rises, and those who exhibit positive temperature coefficient (PTC), which means that they become worse electrical conductors as the temperature rises. Thermistors are widely used as temperature sensors in fire alarms, ovens and refrigerators, digital thermometers and in many automotive applications or Inrush Current Suppression and current-limiting devices for protection of motors and circuits of electrical and electronic devices.

### Summary of the invention

The present invention provides a pocket-size device for the isothermal amplification of nucleic acids which combines the construction simplicity and cost effectiveness of a paper-based antigen test (rapid test) with the accuracy of a molecular test.

The device of the present invention comprises an aperture configured to receive a reaction vessel in which the isothermal nucleic acid amplification takes place. Furthermore, the device comprises an electronic circuit which comprises a positive temperature coefficient (PTC) thermistor as heating element, a power input port and, optionally, a resistor.

### Brief description of the drawings

Figure 1 shows a device according to the present invention.
Figure 2 shows the two parts of a device according to the present invention.
Figure 3 shows the part of a device according to the present invention comprising the thermistor.
Figure 4 shows the part of a device according to the present invention comprising the aperture for receiving the reaction vessel.

### Detailed description of the invention

The present invention provides a pocket-size device for isothermal nucleic acid amplification. The device comprises an aperture configured to receive a reaction vessel in which the isothermal nucleic acid amplification takes place, and an electronic circuit for heating the content of the reaction vessel.

The electronic circuit of the device comprises a PTC thermistor as heating element, a power input port and, optionally, a resistor.

The thermistor is arranged to be thermally coupled to the aperture. Thus, when the reaction vessel is positioned in the aperture, the thermistor is thermally coupled to the reaction vessel.

As electrical current flows though the thermistor, its temperature rises and its resistance also increases, thus self-regulating the passing current. In this way, the temperature of the thermistor is kept constant. Since there is a relation between the applied voltage and the temperature of the thermistor, the temperature is predetermined by the voltage applied to the thermistor.

When the input voltage provided through the power input port is greater than the voltage the thermistor needs to reach the required temperature, the electronic circuit further comprises a resistor, which functions as voltage divider and delivers the desired voltage to the thermistor. Alternatively, it is possible to apply directly the required voltage to the thermistor through the power input port. In this case, the electric circuit does not need to comprise a resistor.

The power input port can have different forms, well known in the art, such as power socket, power plug, power receptacle, molex port, International Electrotechnical Commission (IEC) connector, Japan Solderless Terminal (JST) connector, coaxial port, Deutsches Institut für Normung (DIN) connector, universal serial bus (USB) port, barrel connector, Anderson Powerpole, SAE connector, XLR connector, EmPower connector, Tamiya connector, IP44, or any similar connector that could deliver power. According to a preferred embodiment the power input port comprises a USB port, such as a USB port type A, B, C, or a micro-USB port or any similar port, preferably having a small footprint.

Preferably, the current applied through the power input port is direct current (DC). Preferably, the applied voltage ranges from 1V to 230 V. Preferably, the current up to 5A. Alternatively, alternating current (AC) could also be applied to the device of the present invention. However, and according to PTC thermistor manufacturers, due to the structure of the material, the PTC thermistor on AC voltage is not a purely ohmic resistor. It acts as a capacitive resistor because of the grain boundary junctions. Thus, it is preferred that the input voltage to be used with the device of the present invention is DC voltage. According to a preferred embodiment, the input voltage applied to the device is DC voltage of 5V and the current is 2-3 A.

According to a preferred embodiment, the device does not comprise any additional electronic circuit.

According to another preferred embodiment, the electronic circuit of the device does not comprise any additional electrical component.

The device comprises an aperture configured to receive a reaction vessel in which the isothermal nucleic acid amplification takes place. Preferably, the wall of the aperture is made of a material having thermal conductivity higher than 1 W/(m·K). Examples of such materials include metals, non-metals, or polymers such as high fill epoxy, silicon, aluminum oxide (Al₂O₃), aluminum nitride (AIN), graphene. However, other materials with lower thermal conductivity (for example, 0.01-0.9 W/(m·K)), could be also used, such as polypropylene (PP) and acrylonitrile butadiene styrene (ABS). The aperture and the thermistor are so arranged that the thermistor is thermally coupled to the aperture. Thus, when the reaction vessel is positioned in the aperture, it is thermally coupled to the thermistor. During the operation of the device, as the temperature of the thermistor rises, the temperature of the wall of the aperture also increases thereby heating the reaction vessel and its content.

The remaining portion of the device is preferably made of a material having low thermal conductivity, for example 0.01-0.9 W/(m·K) in order to keep the heat within the aperture of the device. Examples of such materials include ABS, acrylic, PP, FR4, glass, poly (4,4'-oxydiphenylene-pyromellitimide) (such as Kapton^{®}), nylon, plexiglass, polyethylene (PE), polystyrene (PS), rubber, polytetrafluoroethylene (such as Teflon^{®}).

The heating of the reaction vessel occurs mainly through conduction, as the reaction vessel is in thermal contact with the thermistor and the wall of the aperture, and partially through convection as the thermistor heats up any trapped air within the aperture, and the air heats the reaction vessel.

The device may comprise more than one aperture, for example two apertures, each configured to receive a reaction vessel. In such a case more than one amplification reaction can takes place simultaneously. In such an embodiment, preferably, the heating of all vessels is carried out by the same PTC thermistor.

The reaction vessel can be any vessel typically used for the performance of nucleic acid amplification. Preferably, the capacity of the reaction vessel is from 0.1ml to 5 ml. Preferably, the reaction vessel is a tube, such as a PCR tube. The reaction vessel is preferably made of a transparent or translucent material, such as polypropylene.

In order to perform the analysis, the sample to be analyzed and the required reagents, such as primers, enzymes, detection agents are added to the reaction vessel. The reaction vessel is then inserted into the aperture of the device. Then, the power input port is connected to a power source and is kept connected for the required amount of time for the completion of the amplification reaction, typically between 15 and 30 minutes. Then, the reaction vessel is removed from the device and its content is inspected, for example visually, to establish if the nucleic acid of interest was present in the sample. There are a number of different methods for the visual detection of the content of the reaction vessel, which are well known in the art. For example, the visual detection can be based on the color change of the content of the reaction vessel by employing, for example, a pH sensitive dye, or a metal-binding indicator or a DNA intercalating dye. It can be also performed by visualizing the change in the turbidity of the solution upon production of magnesium pyrophosphate. As alternative to naked-eye detection, the production of DNA amplicons can be detected by other methods, such as turbidimetry, fluorimetry or by using a UV light source. A smartphone camera and an appropriate smartphone application may be also used for the sensitive detection of DNA amplification products.

The device of the present invention is used in an isothermal nucleic acid amplification method, i.e. in a method in which the nucleic acid amplification is performed at a constant temperature which is higher than the room temperature. Preferably, the isothermal nucleic acid amplification is LAMP, RPA, RCA or Iso-IMRS. More preferably, the isothermal nucleic acid amplification is LAMP or Iso-IMRS.

The device of the present invention can be disposable, i.e. suitable for one use only, or non-disposable, i.e. suitable for more than one uses.

According to a preferred embodiment, the reaction vessel can be fixed after it is inserted into the aperture of the device, with the use of security means, such as a security ring. In this way, the reaction vessel cannot be inadvertently removed from the device during its operation. After the completion of the amplification, the security means may be removed from the device and then the reaction vessel is removed and inspected.

According to another preferred embodiment, the reaction vessel is fixed with security means, such as a security ring, which is not removable. According to this embodiment, the part of the device comprising the aperture is detachably connected to the part of the device comprising the thermistor and the lower part of the reaction vessel preferably protrudes through the lower surface of the part of the device comprising the aperture. When the analysis of the sample is completed, the part of the device comprising the aperture which holds the reaction vessel is detached from the remaining part of the device and the content of the reaction vessel is inspected to establish if the nucleic acid of interest was present in the sample. In case that the device is non-disposable, the part of the device comprising the aperture is replaced every time the test is completed. In this way, the reaction vessels, which are secured on this part, are disposed properly.

The device of the present invention is pocket-size, i.e. it fits in the palm of an adult user's hand, or in a pocket. Furthermore, it is very simple, low-cost, and easy to manufacture. The heating of the content of the reaction vessel is performed in a simple and efficient manner. Thus, the complex electronic circuits of the devices of the prior art are not needed. Furthermore, the operation of the device is very simple, which means that the user does not need any specialized knowledge or skills to perform the nucleic acid amplification. In addition, the device can be used in any setting and not only in a laboratory.

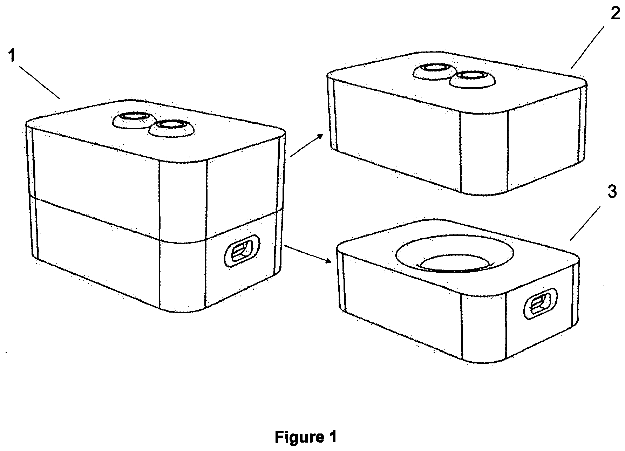

Figures 1 shows an embodiment of a device according to the present invention. The device (1) comprises an upper part (2) which is detachably connected to a lower part (3).

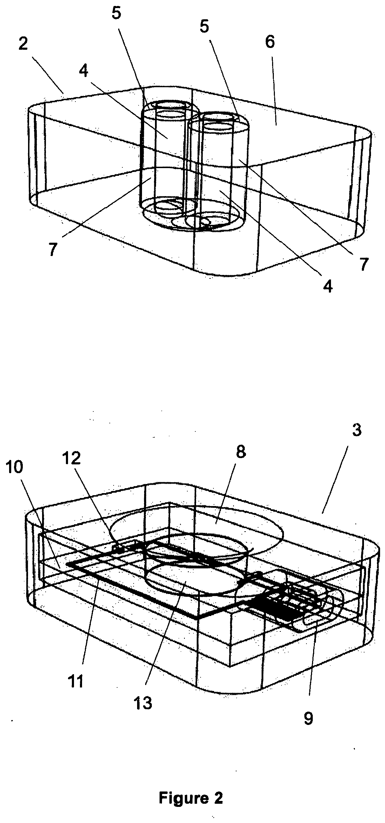

Figure 2 shows that the upper part (2) of the device comprises two apertures/slots (4) for receiving two reaction vessels (not shown). The wall (7) of the aperture (4) which surrounds each reaction vessel is made of aluminium, whereas the remaining portion (6) of the upper part (2) of the device is made of polypropylene. A ring (5) secures each reaction vessel within the opening (4) of the upper part (2). The lower part (3) of the device comprises a circular opening (8) on the side of the thermistor (13) facing the apertures (4), which is combined with a ring (not shown) of the upper part (2) to fix the upper part (2) and thus the apertures (4) in their position. The lower part (3) of the device is made of polypropylene. The lower part (3) further comprises a type-C USB port (9) and an electronic circuit (10) on a PCB. The electronic circuit (10) comprises a conductive line (11), a resistor (12) and a PTC thermistor (13). When the upper part (2) is fixed in its position, the thermistor (13) is thermally coupled to the aperture (4).

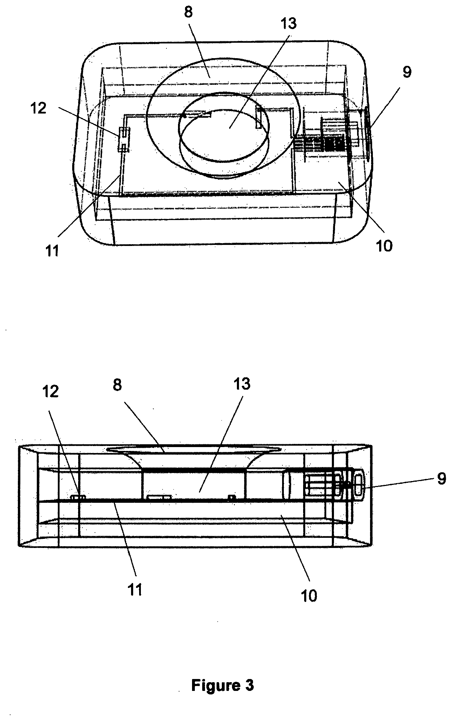

Figure 3 shows different views of the lower part (3) of the device comprising the features mentioned above in relation to Figure 2.

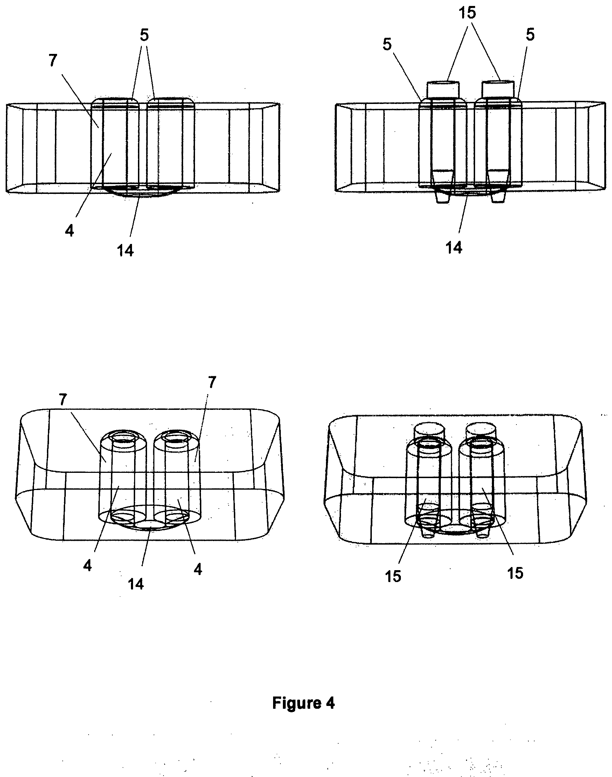

Figure 4 shows different views of the upper part (2) of the device comprising the features mentioned above in relation to Figure 2. Furthermore, Figure 4 shows the ring (14) of the upper part (2) of the device which is combined with the disk shape opening (8) of the lower part (3) of the device. On the right-hand side, the figure shows in addition the reaction vessels (15), which have been inserted into the apertures (4).

### Examples

### Example 1

### COVID-19 disease

The device shown in Figures 1-4 was used for the detection of SARS-CoV-2 RNA from saliva, oropharyngeal or nasopharyngeal swabs without the requirement of nucleic acid purification. Detection of the viral RNA was achieved in less than 20 min after incubation at 65°C using a commercially available set of 6 LAMP primers, an enzyme mix including a reverse transcriptase and a Bst DNA polymerase and a color indicator (phenol red pH indicator or HNB colorimetric dye). The input voltage applied to the device was DC voltage of 5V along with a current of 2.4A. The resistor of the device had a resistance of 4.7Ω.

### Example 2

### Malaria disease

The device shown in Figures 1-4 was used for the detection of *Plasmodium falciparum* DNA from saliva or after DNA extraction from blood samples. Detection of the targeted DNA was achieved in less than 20 min after incubation at 65°C using a set of either 2 Iso-IMRS or 4-6 LAMP commercially available assay primers, a Bst DNA polymerase enzyme and a color indicator (phenol red pH indicator or HNB colorimetric dye). The input voltage applied to the device was DC voltage of 5V along with a current of 2.4A. The resistor of the device had a resistance of 4.7Ω.

## Claims

1. A pocket-size device (1) for isothermal nucleic acid amplification comprising
- an aperture (4) configured to receive a reaction vessel (15) containing a sample of a nucleic acid to be amplified
- an electronic circuit (10), wherein the circuit comprises a heating element for heating the content of the reaction vessel, a power input port (9) and optionally, a resistor (12),
wherein the heating element is a positive temperature coefficient thermistor (13).

2. The pocket-size device (1) for isothermal nucleic acid amplification according to claim 1, wherein the power input port (9) is selected from power socket, power plug, power receptacle, molex port, IEC connector, JST connector, coaxial port, DIN connector, USB port, barrel connector, Anderson Powerpole, SAE connector, XLR connector, EmPower connector, Tamiya connector, or IP44.

3. The pocket-size device (1) for isothermal nucleic acid amplification according to claim 2, wherein the power input port (9) is a USB port.

4. The pocket-size device (1) for isothermal nucleic acid amplification according to any one of the preceding claims, wherein the current applied through the power input port (9) is direct current.

5. The pocket-size device (1) for isothermal nucleic acid amplification according to any one of the preceding claims, wherein the device (1) is made of a material having thermal conductivity from 0.01 W/(m·K) to 0.9 W/(m·K).

6. The pocket-size device (1) for isothermal nucleic acid amplification according to any one of the preceding claims, wherein the wall (7) of the aperture (4) is made of a material having thermal conductivity of at least 1 W/(m·K).

7. The pocket-size device (1) for isothermal nucleic acid amplification according to claim 6, wherein the wall (7) of the aperture (4) is made of a material selected from a metal, a non-metal, high fill epoxy, silicon, aluminum oxide, aluminum nitride, or graphene.

8. The pocket-size device (1) for isothermal nucleic acid amplification according to any one of the preceding claims, wherein the device further comprises means (5) for securing the reaction vessel (15).

9. The pocket-size device (1) for isothermal nucleic acid amplification according to any one of the preceding claims, wherein the isothermal nucleic acid amplification is selected from LAMP, Iso-IMRS, RPA or RCA.

10. The pocket-size device (1) for isothermal nucleic acid amplification according to any one of the preceding claims, wherein the electronic circuit (10) does not comprise any additional electrical component.

11. The pocket-size device (1) for isothermal nucleic acid amplification according to any one of the preceding claims, wherein the device (1) does not comprise any additional electronic circuit.

12. The pocket-size device (1) for isothermal nucleic acid amplification according to any one of the preceding claims, wherein the reaction vessel (15) is tube, preferably a PCR tube.

13. The pocket-size device (1) for isothermal nucleic acid amplification according to any one of the preceding claims, wherein the device (1) comprises a ring (14) at the end of the aperture (4) facing the thermistor (13), which is combined with a circular opening (8) on the side of the thermistor (13) facing the aperture (4), to fix the aperture (4) in its position.

14. The pocket-size device (1) for isothermal nucleic acid amplification according to any one of the preceding claims, wherein the part (2) of the device comprising the aperture (4) is detachably connected to the part (3) of the device comprising the thermistor (13).

15. The pocket-size device (1) for isothermal nucleic acid amplification according to any one of the preceding claims, wherein the device (1) comprises two apertures (4).
